# FASCICULE DE BREVET EUROPEEN

(11) **EP 1 718 673 B1**
(45) Date de publication et mention de la délivrance du brevet: **12.09.2007**
(21) Numéro de dépôt: 05731028.6
(22) Date de dépôt: 23.02.2005
(51) Int. Cl.: C07K 14/76, C07K 14/765

(54) **PROCEDE DE PURIFICATON D' ALBUMINE COMPRENANT UNE ETAPE DE NANOFILTRATION, SOLUTION ET COMPOSITION A USAGE THERAPEUTIQUE LA CONTENANT**
EINEN NANOFILTRATIONSSCHRITT UMFASSENDES VERFAHREN ZUR AUFREINIGUNG VON ALBUMIN, LÖSUNG UND ZUSAMMENSETZUNG ZUR THERAPEUTISCHEN ANWENDUNG, DIE DIESES ENTHALTEN
ALBUMIN-PURIFICATION METHOD COMPRISING A NANOFILTRATION STEP, SOLUTION, AND COMPOSITION FOR THERAPEUTIC USE CONTAINING SAME

(30) Priorité: 27.02.2004 FR 0402001
(43) Date de publication de la demande: 08.11.2006
(73) Titulaire: Laboratoire Français du Fractionnement et des Biotechnologies, 91940 Les Ulis (FR)
(72) Inventeur: BOULANGE, Paul, F-91400 Orsay (FR); CHTOUROU, Sami, F-78990 Elancourt (FR); BOYER, Stéphane, F-91470 Pecqueuse (FR); SCHMITTHAEUSLER, Roland, F-78180 Montigny Le Bretonneux (FR); PADRAZZI, Bruno, F-75014 Paris (FR)
(74) Mandataire: Lhuillier, René
(86) Numéro de dépôt international: PCT/FR2005/000416
(87) Numéro de publication internationale: WO 2005/090402

(56) Documents cités:
- EP-A- 0 498 133
- EP-A- 1 329 460
- EP-A- 1 329 461
- WO-A-96/00237
- BIOLOGICALS., vol. 29, no. 1, 2001, pages 17-25, XP002285689 GB ACADEMIC PRESS LTD., LONDON.

## Description

La présente invention concerne un procédé de purification d'albumine comprenant une étape de nanofiltration, une solution et une composition à usage thérapeutique la contenant, susceptibles d'être obtenues par le procédé de l'invention.

L'albumine est une protéine majeure du plasma sanguin humain ou animal. Les usages cliniques de l'albumine, en tant que principe actif, nécessitent son extraction et sa purification, qui sont classiquement effectuées par des méthodes connues, telles que celles de Cohn et al (J. Am. Chem. Soc., 68, 459, 1946) et Kistler et al. (Vox Sang., 7, 1962, 414-424), applicables de surcroît à une échelle industrielle. EP-A-498 133, EP-A-1 329 461 et EP-A-1 329 460 décrivent des solutions d'albumine non sécurisées WO-A-9 600 237 et Biologicals 29(1), 2001, pages 17-21 décrivent des procédés de purification d'albumine basés sur la mémofiltration.

Les besoins en albumine s'élèvent, selon les pays, à environ 100-300 kg par million d'habitants et il est par conséquent nécessaire, à des fins cliniques, de disposer d'une albumine exempte de virus pathogènes et de tous contaminants, sources de maladies. Ainsi, la sécurité du point de vue des virus transmissibles par transfusion est assurée par des méthodes d'inactivation virale telle que la pasteurisation à l'état liquide d'une composition d'albumine à 60°C pendant 10 heures en présence d'un stabilisant biologiquement compatible (caprylate et/ou tryptophanate de sodium). Cette sécurité sur le plan viral est établie depuis plus de 60 ans et la littérature ne rapporte aucun cas de transmission avérée de virus, par exemple de l'hépatite B, A ou C, ou de VIH. Toutefois, certains auteurs ont mentionné quelques cas relatifs à la présence, après transfusion, de parvovirus B 19 dans des compositions d'albumine ou de produits dérivés de l'albumine. En outre, il a également été rapporté un cas d'une composition d'albumine qui aurait induit la maladie de Creutzfeld-Jacob chez une patiente ayant subi une transplantation hépatique (Créange A., 1995).

Certaines études ont révélé une série d'effets indésirables survenant postérieurement à une transfusion d'albumine attribuée à la présence de substances de nature lipopolysaccharidique (pyrogènes) présentes en quantités inférieures au seuil de détection des méthodes réglementaires, mais pouvant apparaître à l'occasion de la perfusion de grands volumes d'albumine (échanges plasmatiques, par exemple).

D'autres effets secondaires sont liés à la présence de polymères d'albumine apparaissant au cours de sa purification et surtout au cours de l'étape de pasteurisation mentionnée précédemment. D'autres -encore sont liés à la présence de stabilisants ajoutés pour empêcher une dénaturation thermique liée à la pasteurisation et ceci particulièrement chez des patients présentant un terrain allergique.

Afin de pallier le risque de présence d'agents infectieux transmissibles, il a été proposé de produire une albumine dite "recombinante", selon le brevet US 6 210 683 : le gène de l'albumine est introduit dans une cellule-hôte, levure ou bactérie, à potentiel de multiplication élevé. Cette cellule-hôte produit à son tour de l'albumine dans le milieu de culture ou dans son cytoplasme. Celle-ci est ensuite séparée des cellules par extraction et purifiée. Cependant, la présence de protéines de la cellule-hôte est souvent détectée et les méthodes de purification doivent donc être très résolutives, ce qui se fait en général au détriment du rendement. Ainsi, le coût de production d'une albumine recombinante peut alors se révéler trop élevé en comparaison avec celui engendré pour une production d'albumine à partir du plasma.

Une autre possibilité pour contourner les inconvénients énumérés ci-dessus, consiste à mettre en oeuvre des techniques de filtration très largement utilisées pour la rétention virale et de contaminants chimiques, bactériologiques etc. sur filtres qualifiés pour des porosités variables. On peut citer à titre d'exemple l'ultrafiltration qui a été utilisée ponctuellement en l'absence d'autres moyens d'élimination virale pour garantir la sécurité biologique d'une protéine d'extraction, telle que l'hormone de croissance (hGH). La technique requiert un flux tangentiel et n'est destinée qu'à des solutions protéines ou des polypeptides d'un poids moléculaire inférieur ou égal à 65 kDa et à faible concentration. Ceci laisse subsister une probabilité de passage de virus en raison de l'imprécision de fabrication des membranes de filtration. En outre, on observe un colmatage du filtre en fonctionnement frontal aboutissant à un arrêt du flux de filtrat. Le colmatage du filtre est d'autant plus rapide que la concentration en protéine ou polypeptide augmente.

Pour accroître davantage la sécurité virale de solutions biologiques, il est envisageable de mettre en oeuvre la nanofiltration, technique de choix pour la rétention de particules de dimensions supérieures ou égales à 15-20 nm environ. Cette rétention particulaire sur filtres qualifiés, est aisément réalisée à grande échelle pour des solutions aqueuses ou contenant des solutés de faible taille, peptides, acides aminés, ions minéraux ou composés organiques inférieurs à environ 5 kDa.

La nanofiltration de solutés de grande taille a pu être effectuée par exemple pour des facteurs de la coagulation sanguine, le facteur IX et facteur XI. L'article de Burnouf-Radosevich et al (Vox Sang., 67, p. 132-138, 1994 en référence avec Burnouf et al, Vox Sang., 57, p. 225-232, 1989 et Burnouf-Radosevich et al, Transfusion, 22, p. 861-867, 1992) montre que les conditions opératoires de la nanofiltration impliquent de faibles volumes de concentrés de facteur IX et facteur XI (3-4 litres) dont les concentrations en protéines ne dépassent pas 1 g/l (respectivement 0,21 g/l et 0,75 g/l).

Au cours du processus de la nanofiltration, il se produit en effet une accumulation des solutés de grande taille sur le filtre, ce qui aboutit au ralentissement du débit du filtrat jusqu'au colmatage complet du filtre.

Les solutions techniques à ce colmatage consistant à mettre en oeuvre une nanofiltration tangentielle, à augmenter la pression, à changer la direction des flux, ne s'avèrent pas efficace car celui-ci est progressif et irréversible. Un développement à l'échelle industrielle s'en trouve donc fortement compromis, car requérant l'utilisation de très nombreux filtres et des volumes de solutions à filtrer très importants, ce qui engendre un coût prohibitif et des durées de production anormalement accrues.

Pour éviter le phénomène de colmatage, une solution consiste à régler les paramètres physico-chimiques influant sur le rendement de récupération de solutés, tout en évitant le passage de contaminants à travers le filtre. Les variations de ces paramètres, tels que la force ionique, la nature du soluté à filtrer et le pH de la solution à filtrer, ainsi que les conditions opératoires de la filtration, tels que le débit, la pression, ont fait l'objet de très nombreuses études. Celles citées par exemple dans les publications scientifiques de C. Wallis et al, Ann. Rev. Microbiol., 33, p. 413-437, 1979 et S. Jacobs, Methods of Biochemical Analysis, 22, p.307-350, 1974, montrent que l'effet de chaque paramètre pris individuellement peut conduire à une augmentation ou à une diminution de l'efficacité de rétention virale et du rendement de récupération de solutés et que la combinaison de plusieurs paramètres n'est pas, de façon systématique, en faveur d'une synergie des effets d'amélioration des conditions de filtration.

Compte tenu de l'importance que revêt l'albumine sur le plan clinique et des besoins sans cesse croissants, il apparaît nécessaire de mettre à la disposition des patients une albumine présentant un degré de sécurité et de tolérance supérieur à celui des albumines actuellement disponibles, tout en optimisant les conditions de sa production à grande échelle. A cette fin, la Demanderesse s'est donc attachée à mettre au point un procédé de purification de l'albumine comprenant une étape de nanofiltration de solutions d'albumine concentrées répondant à ces objectifs. La présente invention vise ainsi à offrir une solution correspondant à un bon compromis entre les deux critères que constituent l'efficacité de rétention virale et/ou d'autres macromolécules susceptibles d'induire des pathologies ou des effets secondaires chez les patients, et le rendement de récupération de l'albumine, l'importance relative de ces deux critères étant fonction de l'application voulue.

Par conséquent, l'invention concerne un procédé de purification d'albumine caractérisé en ce qu'il comprend une étape consistant à soumettre une solution aqueuse d'albumine, de concentration 15 g/l à 80 g/l et de pH non inférieur à 7, à une nanofiltration dans une plage de température allant de 15°C à 55°C.

Ainsi, la Demanderesse a trouvé de façon surprenante qu'une combinaison judicieuse des valeurs du pH, de la concentration d'albumine, et de la température (donc de la viscosité) de la solution aqueuse d'albumine soumise à l'étape de nanofiltration, permettait d'atteindre une optimisation efficace du rendement de récupération d'albumine et des taux de réduction virale, supérieur à la limite fixée par les autorités de contrôle (4 log) et d'autres macromolécules indésirables. Il a été mis en évidence que la nanofiltration selon les conditions de l'invention permettait la filtration de quantités supérieures à 5 kg d'albumine par m² de filtre, définissant ainsi la charge protéique, tout en optimisant la durée de celle-ci et le débit du filtrat. Les solutions d'albumine nanofiltrées présentent un degré de sécurité très élevé vis-à-vis de contaminants particulaires de taille par exemple d'au moins 13 nm environ, par exemple les virus, tels que les virus non-enveloppés, les prions, les polymères d'albumine (tétramères-décamères) engendrés lors d'étapes de sa fabrication ou au cours de la pasteurisation à 60°C, les lipopolysaccharides en micelles, les acides nucléiques et les protéines agrégées. Les solutions aqueuses d'albumine ainsi obtenues constituent également un produit intermédiaire pouvant être mis en forme pharmaceutique pour les applications cliniques (voir plus loin).

Dans le cadre de l'invention, les solutions aqueuses d'albumine représentent des solutions exemptes de tous réactifs mis en oeuvre lors de différentes étapes classiques de purification ou de fabrication de l'albumine, tels que, par exemple, le polyéthylèneglycol (PEG), l'éthanol, les sels organiques (caprylate de sodium etc.) et inorganiques. Ces divers réactifs sont éliminés de la solution d'albumine par des traitements connus, tels que la diafiltration, l'ultrafiltration, la dialyse etc. Comme indiqué ci-dessus, la présence de tels réactifs, voire même les variations de leur teneur respective d'un échantillon à l'autre, peut aboutir à une efficacité de rétention virale et de rendement de récupération d'albumine défavorables.

Ainsi, la Demanderesse a en outre trouvé de manière surprenante que la diminution de la force ionique était corrélée avec une meilleure réduction virale.

Il convient de préciser que dans le contexte de l'invention, les solutions aqueuses d'albumine soumises à la nanofiltration peuvent toutefois présenter une force ionique inférieure à un seuil maximal lié, dans ce cas, à la valeur du pH basique maximale permise. Cette valeur correspond typiquement à environ 11,5. L'ajustement du pH doit en particulier être réalisé de sorte que la variation de la force ionique engendrée par l'addition d'un ajusteur de pH dans la solution aqueuse d'albumine ne soit que très faible, voire négligeable. De tels ajusteurs de pH seront choisis préférentiellement parmi les bases fortes de métaux alcalins, tels que NaOH et KOH, et parmi les acides forts, tels que HCl, en respectant les critères énoncés ci-dessus. Il est en outre préférable 'd'éviter des ajusteurs de pH à base de composés organiques, tels que des bases organiques.

Ainsi, à titre d'exemple, lorsque les solutions aqueuses d'albumine nécessitent un ajustement du pH jusqu'à la valeur d'environ 11,5, effectué par ajout de NaOH 0,5 M, ces solutions obtenues auront donc une force ionique d'environ 0,0032.

Le procédé de l'invention peut être mis en oeuvre en utilisant tous types de dispositifs de nanofiltration frontale ou tangentielle, en particulier de nanofiltration frontale, connus de l'homme du métier. Actuellement, les filtres utilisés ont une taille de pore inférieure à 100 nm.

L'étape de nanofiltration selon l'invention est de préférence effectuée sur des filtres qualifiés pour une porosité d'au moins 13 nm et représentent, par exemple, les filtres nanométriques de porosité de 15 ± 2 ou 20 ± 2 nm disponibles dans le commerce, se présentant sous la forme de membranes plissées, de membranes planes ou de fibres creuses. A titre d'exemple, on peut citer les nanofiltres en cellulose régénérée tels que PLANOVA® 15 nm et de surface 0,01 m² (provenant de chez Asahi, Japon, ou les filtres à virus PALL (Etats-Unis d'Amérique) donnés pour 20 ou 50 nm.

On peut utiliser toutes sources de matière première d'albumine (produit pur lyophilisé, concentré etc.) et notamment celles issues du fractionnement du plasma sanguin humain ou animal par les méthodes de Cohn et al (J. Am. Chem. Soc., 68, 459, 1946) ou Kistler et al. (Vox Sang., 7, 1962, 414-424).

Le pH de la solution aqueuse d'albuminé est de préférence situé dans la plage de valeurs allant d'environ 7,8 à environ 11,5, et, de façon plus préférée, de 9 à 10,5. Des valeurs de pH supérieures à environ 11,5 dénaturent de façon irréversible l'albumine.

Le procédé de l'invention est de préférence mis en oeuvre avec des solutions aqueuses d'albumine de concentration située dans la plage allant de 40 à 60 g/l et dans une plage de températures allant de 30 à 55°C.

Selon l'invention, le procédé peut en outre comprendre une étape d'ajout d'un sel ou mélange de sels pharmaceutiquement acceptables dans la solution aqueuse d'albumine pour obtenir une solution de force ionique supérieure à 0.0032 et, de préférence, située dans la plage allant de 0,01 à 0,55, plus préférentiellement, de 0,01 à 0,3, de façon encore plus préférentielle, de 0,05 à 0,15, et, en particulier, de 0,1 et 0,13. On utilise préférentiellement les sels de métaux alcalins et, en particulier, le chlorure de sodium présent en quantité conférant à la solution d'albumine une force ionique de 0,15.

Bien que toute albumine convienne comme matière première, son origine peut toutefois constituer un facteur jouant un rôle sur le rendement de la nanofiltration selon qu'elle contienne ou non de stabilisants thermiques ou qu'elle ait été traitée ou non par la chaleur (choc thermique ou pasteurisation). Ainsi, l'utilisation dans le procédé selon l'invention d'une albumine obtenue par extraction éthanolique selon Cohn et al ou Kistler *et al* citées ci-dessus, et purifiée par chromatographie d'échange d'ions ou d'affinité, peut donner lieu à un accroissement du rendement de récupération de l'albumine et/ou une diminution de la durée de filtration.

La nanofiltration de la solution aqueuse d'albumine peut être effectuée en deux étapes successives sur respectivement deux filtres de porosité décroissante. Avantageusement, ces deux étapes successives sont effectuées respectivement sur des filtres de porosité de 23 à 50 nm et 15 ou 20 nm.

Lorsque des filtres PLANOVA^{®} 15 nm et de surface 0,01 m² sont utilisés, le procédé de l'invention est mis en oeuvre à des pressions non supérieures à 1 bar, celles-ci étant de préférence situées dans la plage allant de 0,2 à 0,8 bar.

Le procédé peut comprendre une étape ultérieure de traitement spécifique connu destiné à rendre les solutions aqueuses d'albumine appropriées à divers usages thérapeutiques conformément à la Pharmacopée européenne, tel que leur ajustement au pH physiologique le cas échéant, à l'isotonie dans le cas d'une injection intraveineuse et à une concentration saline physiologiquement acceptable, et/ou de conditionnement par exemple sous forme liquide ou lyophilisée.

L'invention concerne également une solution aqueuse d'albumine viralement sécurisée susceptible d'être obtenue par la mise en oeuvre du procédé de l'invention, dans laquelle les sites de transport et de fixation de principes actifs thérapeutiques de l'albumine sont disponibles. Cette solution d'albumine est en outre caractérisée en ce qu'elle est exempte de macromolécules d'une constante de sédimentation, selon Svedberg Ph. et al (Ultracentrifüg, 7ème édition, Ed. Steinkopff, Dresden, 1940), supérieure à 7 S (soit de masse moléculaire d'environ 160 kDa). En particulier, cette solution d'albumine contient au plus 1% de polymères d'albumine de taille inférieure à 100 nm, de préférence inférieure à 20 nm.

L'invention concerne en outre une composition d'albumine à usage thérapeutique obtenue par un traitement d'adaptation de ladite solution d'albumine selon l'invention à un usage clinique.

La sécurisation, obtenue pour ces compositions d'albumine à usage thérapeutique permet de supprimer l'étape de pasteurisation source d'inconvénients énumérés plus haut et donc l'adjonction de stabilisants habituels de protection contre les effets thermiques, qui se fixent en outre sur les sites de l'albumine empêchant par conséquent l'albumine de fixer des molécules d'intérêt. L'albumine de ces compositions selon l'invention conserve donc son potentiel de fixation et de transport de divers principes actifs et atténue par cette liaison leur toxicité ou en augmente la biodisponibilité par un effet retard.

On peut également citer d'autres applications pour lesquelles les compositions d'albumine selon l'invention peuvent être proposées:
- stabilisation vis-à-vis de la dénaturation et de l'oxydation de protéines de faible concentration (inférieure au g/l) et d'activité spécifique élevée, telles que le facteur VIII, lé facteur von Willebrand et leur équivalent recombinant, d'immunoglobulines spécifiques, d'anticorps monoclonaux, de vaccins (viraux, bactériens, protéiques, ADN), d'allergènes pour la détection d'allergies chez des patients sensibles, de cytokines ou encore d'hormones peptidiques ;
- excipient pour milieu de propagation et d'incubation pour les fécondations "in vitro" d'ovocytes humains ; et
- protéines standard témoin pour servir de placebo dans les études cliniques.

L'invention sera mieux comprise à l'aide des exemples qui suivent, en référence aux dessins annexés, sur lesquels:
- la figure 1 est une illustration d'un dispositif de mise en oeuvre du procédé de l'invention, et
- les figures 2 à 6 sont des tracés des variations du débit instantané de filtration (ml/min) en fonction de la durée de nanofiltration, obtenus en fonction de différents paramètres.

### Exemple 1

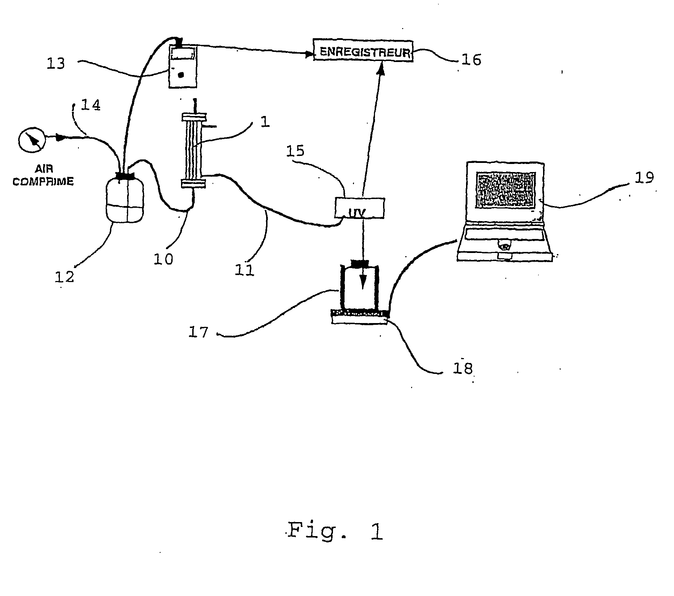

### 1.1 Méthodologie de mise en oeuvre du procédé de nanofiltration (figure 1)

Un dispositif de filtration 1 comprenant un filtre PLANOVA® 15 nm et de surface 0,01 m² (provenant de chez Asahi, Japon) est équipé de tuyaux 10, 11 en sortie de rétentat (solution avant filtration) et en entrée et sortie de filtrat (solution après filtration), en matériaux compatibles sur le plan pharmaceutique, de diamètres d'environ 5 mm, fermés par des pinces à clamper. Le dispositif est placé sur un statif (non représenté) en position verticale à l'aide de pinces.

L'entrée du filtre de 15 nm est raccordée, par le tuyau 10, à un récipient pressurisable 12 dont la pression' est mesurée grâce à un manomètre numérique 13 branché sur le circuit amont du filtre.

Avant utilisation, un test d'intégrité est mis en oeuvre sur le filtre de 15 nm suivant la procédure définie par le fabricant : "Test de fuite à l'air lors de test d'intégrité pré et post filtration des filtres PLANOVA° 15, 35, 75 nm".

On procède ensuite au rinçage de l'installation. A cet effet, l'arrivée d'air comprimé 14 est branchée sur le réservoir pressurisable 12 rempli d'un volume d'environ 100 ml de NaCl à 9 g/l. Le flacon est pressurisé progressivement jusqu'à obtenir 0,5 bar à l'entrée du filtre de 15 nm. Le filtre de 15 nm est rempli en purgeant l'air par la sortie rétentat, sans remplir l'extérieur des fibres constituant le filtre. La sortie basse est ouverte et est reliée à une cellule de détection 15 de mesure de la densité optique connectée à un enregistreur 16, la sortie filtrat haute restant clampée. Le filtrat de rinçage est recueilli dans un récipient 17 sur une balance 18 reliée et pilotée par un microordinateur 19 qui enregistre l'augmentation du poids de filtrat, ce qui permet de suivre le débit instantané de filtration. Le temps nécessaire pour filtrer un volume minimum de 40 ml à 0,5 bar est mesuré, puis en on déduit le débit moyen en ml/min. Le montage est dépressurisé progressivement et toutes les sorties sont clampées. Selon la température de la nanofiltration voulue, l'installation peut être placée à température ambiante (environ 20°C) ou dans une étuve dont la température d'utilisation est comprise entre 25 et 60°C.

### 1.2 Solutions d'albumine

On utilise comme solutions aqueuses d'albumine de référence, des solutions à 20 g/l, à pH 7, comprenant du NaCl à 9 g/l. La matière première d'albumine utilisée, notée A, hormis pour les exemples 6 et 9 ci-après, est celle obtenue par fractionnement du plasma humain selon Kistler et al ayant ensuite subi un traitement d'élimination de l'alcool, de diafiltration et de concentration par ultrafiltration. Il convient de préciser que le fractionnement ci-dessus de diverses sources de plasma conduit généralement à une albumine, dont les caractéristiques peuvent varier en raison de la nature biologique de la matière première. Par conséquent, celle-ci peut donc influer sur la durée de nanofiltration, le rendement etc.

On prépare des solutions aqueuses d'albumine à filtrer, à partir des solutions de référence ci-dessus, par modification de leurs caractéristiques telles que la concentration, le pH et la force ionique. Leurs volumes sont ajustés de façon à obtenir les charges protéiques voulues. Selon les exemples, les concentrations en albumine varient de 15 g/l à 80 g/l. L'ajustement du pH est effectué par ajout de NaOH ou de HCl 0,5 M et celui de la force ionique, par ajout de chlorure de sodium. Toutes les solutions d'albumine sont ensuite préfiltrées sur filtres de 0,2 µm disponibles dans le commerce.

### 1.3 Nanofiltration d'une solution d'albumine

Une fois la filtration d'un volume prédéterminé d'albumine achevée, l'entrée du filtre est fermée. Un volume d'eau purifiée pour injection (PPI) de rinçage est introduit dans le réservoir 12 remis sous pression et le filtre est purgé. Le filtrat est recueilli jusqu'à diminution sensible de la densité optique suivie sur l'enregistreur 16. Le circuit aval est ensuite purgé afin de recueillir la fin du filtrat. Des prélèvements sont effectués sur le filtrat et sont soumis à des contrôles analytiques ultérieurs, tels que les dosages en polymères, le titre viral etc. On mesure aussi la durée de filtration nécessaire d'un volume de solution d'albumine de façon à obtenir une charge protéique fixée et le rendement de filtration représenté par le quotient entre la quantité en albumine du filtrat et celle du rétentat.

Après utilisation, un test d'intégrité est mis en oeuvre sur le filtre de 15 nm suivant la procédure définie par le fabricant : "Test de fuite à l'air lors de test d'intégrité pré et post filtration des filtres PLANOVA® 15, 35, 75 nm".

### Exemple 2

On effectue deux essais préliminaires destinés à évaluer la faisabilité de la nanofiltration, en termes de charge protéique, de durée et de rendement, lorsque celle-ci est mise en oeuvre avec des solutions d'albumine A (exemple 1), filtrées à 20°C et sous une pression de 0,5 bar. Des volumes de solutions A sont préparés pour une charge protéique de 0,5 kg/m² et 1 kg/m². Les résultats sont montrés dans le Tableau 1.

**Tableau 1**

| Solutions | Charge protéique (kg/m²) | Durée de filtration (min) | Rendement protéique (%) |
|---|---|---|---|
| A | 0,5 | 190 | 98 |
| A | 1 | 282 | 99 |

Ces résultats montrent que selon les conditions fixées, la nanofiltration de solutions d'albumine A permet une charge protéique de 1 kg/m², sans colmatage du filtre, et avec un excellent rendement. On observe également que le fait de doubler la charge protéique n'entraîne pas une durée de nanofiltration deux fois plus importante.

Les résultats de ces essais ont donc incité la Demanderesse à envisager la nanofiltration de solutions d'albumine de façon à obtenir des charges protéiques plus importantes en optimisant le cas échéant certains paramètres influant sur celle-ci.

### Exemple 3

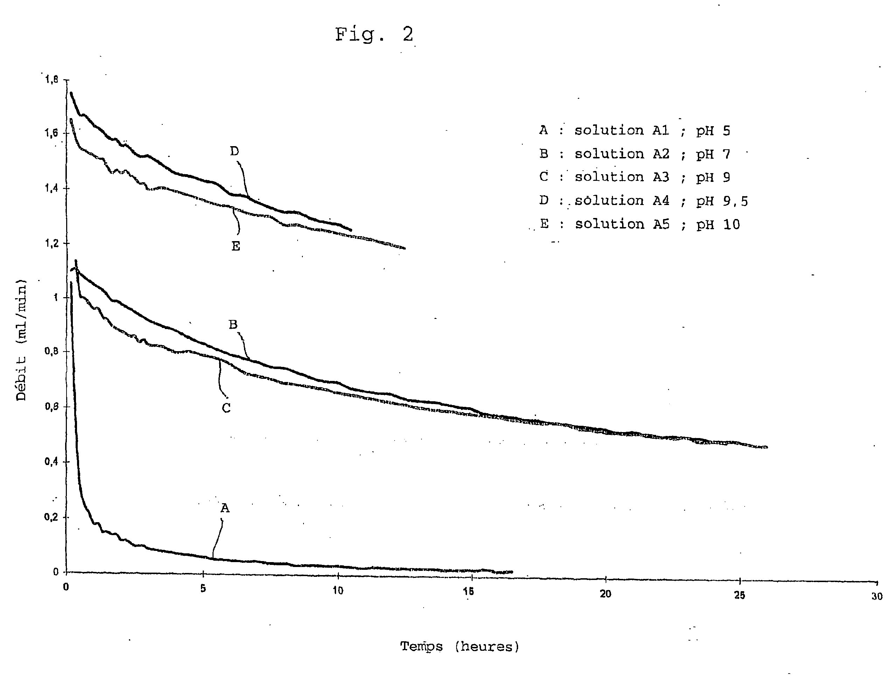

L'influence du pH des solutions d'albumine sur les durées de filtration et les rendements pour obtenir une charge protéique de 4 kg/m² est établie en considérant, comme matière première, un lot d'albumine A. Cinq solutions d'albumine à 40 g/l, notées A1 à A5, sont préparées dans une solution de NaCl à 9 g/l puis respectivement ajustées à un pH de 5, 7, 9, 9,5 et 10 par des solutions de HCl 0,5 M ou de NaOH 0,5 M, et sont soumises à une nanofiltration. Les essais de nanofiltration sont effectués à 20°C sous une pression de 0,5 bar. La figure 2 montre les courbes de débit de filtration (ml/min) en fonction de la durée de la nanofiltration pour chacune des solutions considérées.

L'analyse de ces courbes montre que pour la solution A1 (pH 5), il se produit une diminution rapide du débit et, par conséquent, un colmatage du filtre. Pour les solutions A2 et A3, on constate une diminution du débit initial plus rapide pour la solution A3, mais avec des variations tout à fait acceptables, sans l'observation d'un colmatage après plus de 25 heures de filtration. Les meilleurs résultats sont obtenus pour les solutions A4 et A5, c'est-à-dire à pH 9,5 et 10, avec des débits initiaux respectifs plus élevés et des durées de filtration plus faibles que pour celles des solutions A2 et A3, la solution A4 présentant un débit initial le plus optimal.

### Exemple 4

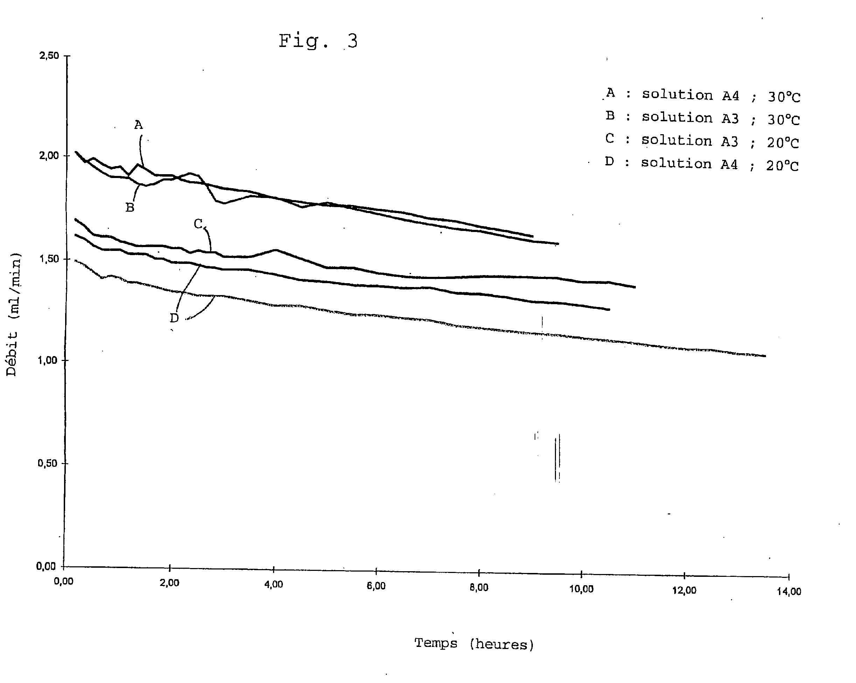

Dans cet exemple, l'effet conjoint du pH et de la température de façon à obtenir une charge protéique de 4 kg/m² est testé sur les solutions A3 et A4, définies à l'exemple 3, à 20°C et à 30°C. Les essais de nanofiltration sont effectués sous une pression de 0,5 bar. Les durées de filtration ainsi obtenues sont indiquées au Tableau 2. La figure 3 rassemble les courbes de débit de filtration (ml/min) en fonction de la durée de la nanofiltration pour chacune des solutions considérées.

**Tableau 2**

| Solution | Température (°C) | Durée de filtration (h) | rendement (%) |
|---|---|---|---|
| A3 | 20 | 11 | 98 |
| A3 | 30 | 9,5 | 98 |
| A4* | 20 | 12,75 ± 0,75 | 98 |
| A4 | 30 | 9 | 98 |

| | | | |
|---|---|---|---|
| * : deux essais de nanofiltration | | | |

A 30°C, on obtient les durées de filtration les plus faibles avec des courbes de débit pratiquement superposables (solutions A3 et A4) sans l'observation d'un colmatage progressif du filtre. A 20°C, les débits de filtration sont un peu plus faibles qu'à 30°C.

### Exemple 5

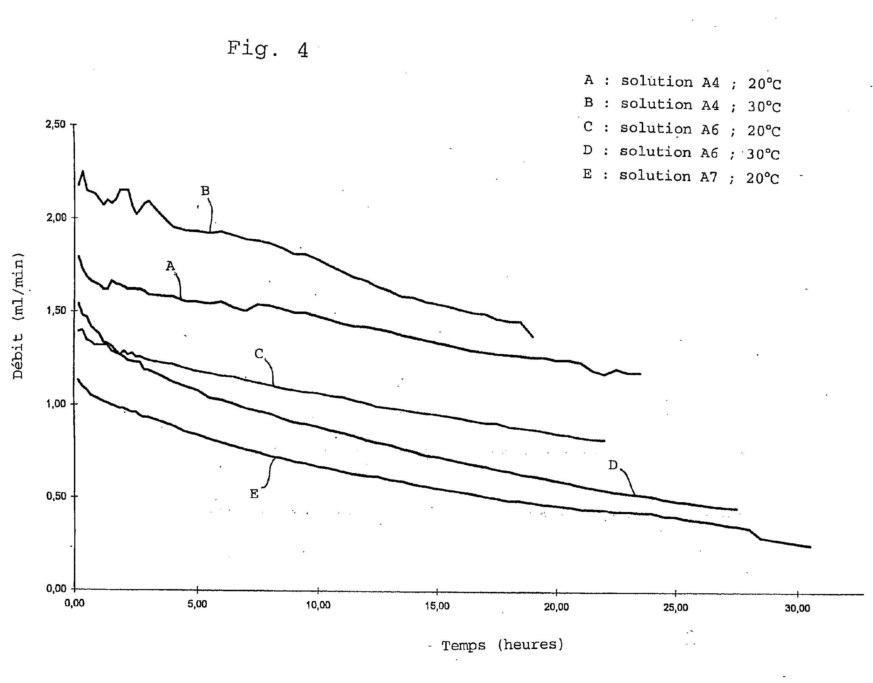

Cet exemple est destiné à montrer l'influence conjointe de la concentration en albumine et de la température sur la nanofiltration pour obtenir une charge protéique de 8 kg/m², en analysant les durées de filtration obtenues (Tableau 3) et les courbes de débit (figure 4). Pour cette série d'essais, on considère les solutions A4 (40 g/l), A6 (60 g/l) et A7 (80 g/l), toutes trois comprenant du NaCl à 9 g/l, pH 9,5, qui sont ensuite soumises à une nanofiltration effectuée sous une pression de 0,5 bar.

**Tableau 3**

| Solutions | Température (°C) | Durée de filtration (h) | Rendement (%) |
|---|---|---|---|
| A4 | 20 | 24,6 | 98 |
| A4 | 30 | 19,5 | 98 |
| A6 | 20 | 22,5 | 98 |
| A6 | 30 | 28,4 | 98 |
| A7 | 20 | 31 | 98 |

L'analyse des résultats du Tableau 3 et de la figure 4 montre que l'on peut charger le filtre par 8 kg/m² sans colmatage. En particulier, les conditions optimales de filtration sont obtenues pour la solution A4 à 30°C. Pour la solution A6, le débit initial de filtration à 30°C est plus grand qu'à 20°C avec en outre une durée de filtration plus longue. Ces résultats ont donc incité à considérer pour la solution la plus concentrée A7 une température de nanofiltration de 20°C. Dans ces conditions, la durée de filtration est la plus longue et le débit initial le plus faible.

### Exemple 6

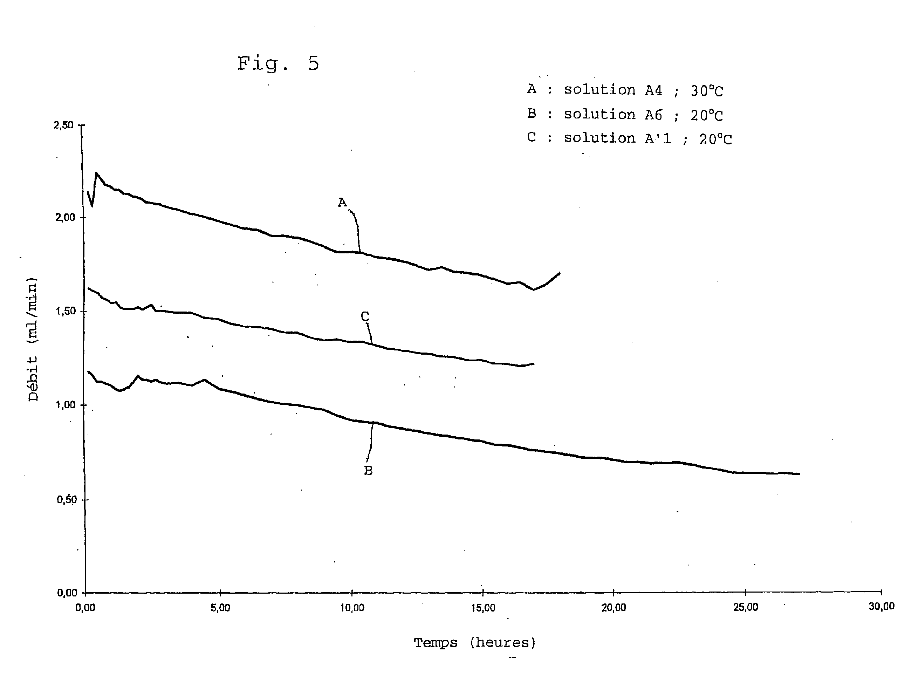

Trois essais de nanofiltration sont effectués avec des solutions préparées à partir de deux matières premières d'albumine différentes. La première représente l'albumine A et la deuxième, l'albumine A purifiée par chromatographie sur colonne échangeuse d'ions (dénommée A'). Pour cette série d'essais, on considère les solutions A4 et A6 de l'exemple 5, et A'1 (60 g/l, NaCl à 9 g/l, pH de 9,5) qui sont ensuite soumises à une nanofiltration effectuée à 20 et 30°C, sous une pression de 0,5 bar et de façon à obtenir une charge protéique de 8 kg/m². Le Tableau 4 montre les durées de filtration et le rendement. La figure 5 montre les courbes de débit de filtration (ml/min) en fonction de la durée de la nanofiltration pour chacune des solutions considérées.

**Tableau 4**

| Solutions | Température (°C) | Durée de filtration (h) | Rendement (%) |
|---|---|---|---|
| A4 | 30 | 18,5 | 98 |
| A6 | 20 | 27,5 | 98 |
| A'1 | 20 | 17 | 98 |

La durée de la nanofiltration pour A'1 est la plus courte. Cependant, lorsqu'on opère avec la solution A4, on obtient le débit initial le plus optimal.

### Exemple 7

L'effet de la variation de la force ionique de solutions d'albumine sur les durées de filtration et les rendements est étudié pour trois solutions d'albumine A à 40 g/l, respectivement préparées dans de l'eau purifiée pour injection (solution A8), dans une solution de NaCl à 9 g/l (solution A2 ; force ionique 0,15) et de NaCl à 30 g/l (solution A9 ; force ionique 0,5), toutes trois étant à pH 7. Les essais de nanofiltration sont effectués à 20°C sous une pression de 0,5 bar, de façon à obtenir une charge protéique constante de 4 kg/m². Les résultats obtenus sont rassemblés dans le Tableau 5.

**Tableau 5**

| Solutions | Concentration en NaCl (g/l) | Durée de filtration (h) | Rendement (%) |
|---|---|---|---|
| A8 | 0 | 24,5 | 98 |
| A2 | 9 | 18,7 | 98 |
| A9 | 30 | 22 | 98 |

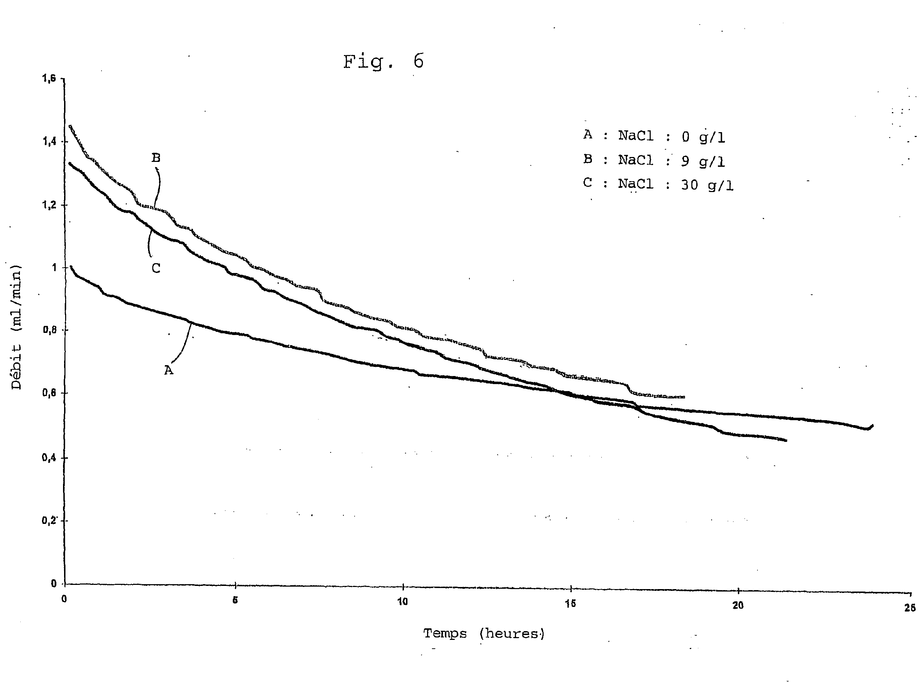

Le meilleur résultat en termes d'optimisation de la durée de filtration et du rendement est obtenu pour une force ionique de 0,15, proche de celle correspondant à l'isotonie. Ce résultat est confirmé par un suivi de l'augmentation de poids filtré qui permet le tracé des variations du débit de filtration (ml/min) en fonction de la durée de la nanofiltration des solutions étudiées (Figure 6). Dans cet exemple, on constate que l'augmentation de la concentration en NaCl jusqu'à 30 g/l ne diminue pas le temps de filtration.

### Exemple 8

Cet exemple est destiné à illustrer l'influence des variations de la charge protéique et de la pression de nanofiltration sur les durées de filtration et les rendements en produits obtenus. A cette fin, on considère les deux solutions d'albumine A (Exemple 2) et A2 (pH 7). Les résultats figurent au Tableau 6.

**Tableau 6**

| Solutions (g/l) | Charge, protéique (kg/m²) | Pression (bar) | Durée de filtration (min) | Rendement (%) |
|---|---|---|---|---|
| A2 | 2 | 0,5 | 355 | 100 |
| A | 2 | 0,8 | 457 | 98 |
| A2 | 4 | 0,5 | 1350 | 98 |
| A2 | 4 | 0,8 | 1730 | 98 |

Ces résultats indiquent que pour les solutions A et A2, une augmentation de la pression engendre des durées de filtration plus élevées et ceci malgré le fait que la solution A soit moins concentrée que la solution A2. Les résultats obtenus pour les solutions A2 montrent que les filtres PLANOVA^{®} 15 nm présentent une capacité ou charge protéique de 4 kg/m² et qu'une augmentation de pression ne diminue pas le temps de filtration.

### Exemple 9

Cet exemple est destiné à montrer la possibilité de mise en oeuvre de la nanofiltration à l'échelle industrielle en utilisant des filtres PLANOVA^{®} 15 nm de 1 m² de surface. L'albumine utilisée est obtenue par fractionnement selon la méthode de Kistler *et al* et est rendue de qualité thérapeutique par chauffage à 60°C pendant 10 h en présence d'un stabilisant adéquat, selon les exigences de la Pharmacopée européenne. L'albumine B ainsi obtenue est ensuite mélangée dans un tampon comprenant : 0,01 M de citrate trisodique, 0,12 M de glycine, 0,016 M de L-lysine, 0,001 M de chlorure de calcium et 0,17 M de chlorure de sodium, dont le pH vaut 7-7,5. La concentration finale des solutions d'albumine est de 20 g/l. Diverses solutions ainsi obtenues, après avoir été préfiltrées sur filtres de 0,2 µm, sont soumises à une nanofiltration décrite à l'exemple 1, à température ambiante et sous une pression de 0,5 bar. Les volumes de solution B percolés à travers le filtre sont déterminés de façon à obtenir une charge protéique de 120 g/m². Les mêmes essais de nanofiltration sont effectués en considérant des filtres de 0,01 m². Le Tableau 7 rassemble les résultats de durées de filtration et de rendement obtenus pour les deux filtres, et ceux-ci constituent les valeurs moyennes de 3 essais.

**Tableau 7**

| Solutions B | Durée de la filtration (h) | Rendement (%) |
|---|---|---|
| Filtre de 0,01 m² | 3,1 ± 0,1 | 87 ± 4 |
| Filtre de 1 m² | 2,5 ± 0,1 | 83 ± 1 |

Les résultats obtenus montrent que l'utilisation de filtres de 1 m² de surface conduit à une durée de filtration plus faible par rapport à celle obtenue avec des filtres de 0,01 m² de surface mais avec une très légère baisse du rendement de filtration.

### Exemple 10

Les mêmes solutions B et conditions opératoires de l'exemple 9 sont reprises dans le but de mesurer la réduction du taux de polymères après la nanofiltration (Tableau 8).

**Tableau 8**

| Solutions B | Taux de polymères avant filtration (%) | Taux de polymères après filtration (%) |
|---|---|---|
| Filtre de 0,01 m² | 3,1 | 0,47 ± 0,13 |
| Filtre de 1 m² | 4,3 ± 0,7 | 0,38 ± 0,05 |

Il convient de noter que le traitement de pasteurisation de l'albumine engendre des polymères dont le pourcentage reste toutefois inférieur à la limite de 5% préconisée par la Pharmacopée européenne (4ème édition, chapitre "solution d'albumine humaine", page 642). Les essais de nanofiltration effectués sur les deux surfaces de filtres considérés, permettent la réduction du taux de polymères d'un facteur d'environ 10 dans les deux cas.

### Exemple 11

Afin de montrer l'influence de la variation des conditions opératoires de mise en oeuvre de la nanofiltration (surface du filtre, pression et température) et des paramètres physico-chimiques de solutions d'albumine (pH, composition du tampon) sur le taux de réduction virale, des virus ont été introduits dans différentes solutions d'albumine A (20 g/l) qui ont été ensuite soumises à des essais de nanofiltration. Les essais sont effectués en infectant les solutions d'albumine par du virus bactériophage Phi-X 174, dont les suspensions sont réalisées selon la norme AFNOR NFT 72-181 (décembre 1989). Ce virus constitue un bon marqueur du filtre de 15 nm, sa taille étant comprise entre 25 et 30 nm, ce qui correspond aux virus non enveloppés transmissibles à l'homme tels que le parvovirus B19 dont l'inactivation par pasteurisation n'est pas satisfaisante.

Les résultats obtenus sont montrés au Tableau 9.

**Tableau 9**

| Surface du filtre (m²) | Pression (bar) | T (°C) | Osmolalité (mosm) | Composition du tampon | pH | Réduction virale (Δ log) |
|---|---|---|---|---|---|---|
| 0,01 | 0,5 | 35 | 280 | NaCl : 0,15 M | 7,2 | 5,9 |
| 0,001 | 0,5 | 35 | 280 | NaCl : 0,15 M | 7,2 | 6,5 |
| 0,001 | 0,2 | 35 | 280 | NaCl : 0,15 M | 7,2 | > 5,0 |
| 0,001 | 1,0 | 35 | 280 | NaCl : 0,15 M | 7,2 | 4,9 |
| 0,001 | 0,5 | 26 | 280 | NaCl : 0,15 M | 7,2 | > 5,5 |
| 0,001 | 0,5 | 35 | 280 | NaCl : 0,15 M | 4,1 | > 6,0 |
| 0,001 | 0,5 | 35 | 555 | NaCl : 0,30 M | 7,2 | 4,2 |
| 0,001 | 0,5 | 35 | 610 | NaCl : 0,15 M ; | 7,2 | > 6,3 |
| | | | | Saccharose : 100 g/l | | |
| 0,001 | 0,5 | 35 | 335 | Na₂SO₄ 0, 15 M | 7,2 | 3,7 |
| 0,001 | 0,5 | 35 | 280 | NaCl : 0,15 M | 7,2 | > 6,5 |
| | | | | Tween 80 : 0,06 % | | |

L'analyse de ces résultats montre pour les variations des conditions opératoires que :
- la seule variation de la surface du filtre et celle de la température, quelque soit la solution d'albumine considérée, n'ont quasiment pas d'influence sur le taux de réduction virale;
- une augmentation de la pression de 0,5 bar à 1,0 bar a une influence défavorable sur le taux de réduction virale qui reste toutefois élevé (> 4 log).

Les variations des paramètres physico-chimiques des solutions d'albumine A montrent que:
- la présence d'un détergent (Tween^{®} 80) et d'un composé non ionique (le saccharose), et une variation du pH, n'ont quasiment pas d'influence sur le taux de réduction virale ;
- en revanche, le remplacement du NaCl par un sel divalent, le sulfate de sodium, a une influence négative sur ce taux qui devient inférieur au seuil autorisé par les autorités de contrôle. Le même phénomène est observé lorsqu'on augmente la concentration en NaCl (de 0,15 M à 0,3 M) bien que le taux de réduction soit satisfaisant.

### Exemple 12

Les propriétés de l'albumine A nanofiltrée, sans stabilisant, dans le transport et la fixation de médicaments, ont été étudiées en les comparant avec celles obtenues pour deux lots différents d'albumine A pasteurisée en présence de caprylate de sodium.

On dispose, à cet effet, de trois solutions A4 (exemple 3) dont une est nanofiltrée dans les conditions de l'invention. Celles-ci sont ensuite mises en forme pharmaceutique dans un tampon phosphate 0,07 M, pH 7,4, de façon à obtenir des compositions d'albumine, notées A'4, A"4 et A'''4, de concentration de 2,5 g/l. Les compositions d'albumine A"4 et A'''4, non nanofiltrées, ont été respectivement pasteurisées en présence de caprylate de sodium. On prélève un échantillon de 1 ml de chacune des compositions et on y ajoute respectivement un volume compris entre 10 µl et 1000 µl d'une solution alcoolique mère de [¹⁴C]warfarine et de [¹⁴C] diazepam à 0,1 M, deux principes actifs de la classe respective des anticoagulants et des neurotropes, de façon à obtenir des concentrations variables en ces principes actifs dans les compositions d'albumine considérées. L'ensemble est ensuite homogénéisé.

L'étude des propriétés de l'albumine dans la fixation des deux principes actifs ci-dessus est basée sur la méthodologie de la dialyse à l'équilibre, connue de l'homme du métier, dont le principe est résumé ci-après. On se procure un dispositif de dialyse comportant une cellule avec deux compartiments séparés par une membrane de dialyse appropriée. On introduit un volume V d'un mélange albumine/principe actif dans le compartiment 1 et dans le compartiment 2, on introduit le même volume V de tampon de dialyse (tampon phosphate défini ci-dessus). Une fois l'équilibre atteint, au bout de quelques heures, le compartiment 1 comprend le principe actif non lié et celui lié à l'albumine et le compartiment 2, le principe actif non lié. Des échantillons sont prélevés de chaque compartiment 1 et 2 et la radioactivité est mesurée par scintillation liquide. Ces mesures permettent de déterminer les concentrations respectives en principes actifs liés et non liés à l'albumine.

Le Tableau 10 donne les résultats des pourcentages de [¹⁴C]diazepam lié à l'albumine obtenus en considérant respectivement les compositions A'4, A"4 et A'''4 dans lesquelles des volumes croissants en principe actif ont été ajoutés. Le Tableau 11 présente les résultats obtenus en considérant la [¹⁴C] warfarine à la place du [¹⁴C]diazepam. Les résultats représentent la valeur moyenne de 5 essais.

**Tableau 10**

| Compositions d'albumine | Concentration totale du [¹⁴C] diazepam (µm) | Pourcentage de [¹⁴C]diazepam lié à l'albumine |
|---|---|---|
| A'4 | 1,68 ± 0,06 | 80,9 ± 1,46 |
| | 4,17 ± 0,28 | 82,34 ± 1,92 |
| | 8,04 ± 0,24 | 78,08 ± 1,83 |
| | 40,09 ± 1,28 | 57,18 ± 2,14 |
| | 65,27 ± 3,46 | 48,58 ± 1,49 |
| | 115,02 ± 4,83 | 36,38 ± 1,69 |
| A"4 | 1,09 ± 0,23 | 34,46 ± 2,04 |
| | 5,52 ± 0,53 | 32,26 ± 1,34 |
| | 21,95 ± 0,42 | 31,00 ± 0,93 |
| | 42,84 ± 5,67 | 31,85 ± 2,99 |
| | 85,00 ± 13,6 | 30,12 ± 2,00 |
| | 109,79 ± 10,96 | 29,12 ± 2,67 |
| A'''4 | 1,23 ± 0,09 | 38,47 ± 1,88 |
| | 2,91 ± 0,21 | 40,93 ± 4,83 |
| | 11,55 ± 0,19 | 37,85 ± 1,25 |
| | 44,98 ± 3,14 | 34,96 ± 0,6 |
| | 86,09 ± 5,01 | 33,04 ± 1,17 |
| | 107,97 ± 9,43 | 30,74 ± 1,78 |

Les résultats montrent que plus la concentration en [¹⁴C]diazepam augmente dans la composition d'albumine A'4, plus le pourcentage de [¹⁴C]diazepam lié à l'albumine diminue. Ceci met évidence la présence dans l'albumine d'un site ou liaison saturable et d'une liaison non saturable.

L'augmentation de la concentration en [¹⁴C]diazepam dans les compositions d'albumine A"4 et A'''4, conduit à des pourcentages de [¹⁴C] diazepam lié à l'albumine sensiblement constants. Le site de fixation identifié pour l'albumine A nanofiltrée sans stabilisants de la composition A'4 n'est donc plus fonctionnel car occupé par le stabilisant.

**Tableau 11**

| Compositions d'albumine | Concentration totale de la [¹⁴C] warfarine (µm) | Pourcentage de la [¹⁴C]warfarine liée à l'albumine |
|---|---|---|
| A'4 | 1,99 ± 0,19 | 93,32 ± 0,49 |
| | 10,12 ± 0,71 | 91,75 ± 0,75 |
| | 34,29 ± 2,28 | 83,43 ± 1,11 |
| | 62,73 ± 3,55 | 71,74 ± 1,20 |
| | 103,66 ± 4,24 | 55,34 ± 2,94 |
| | 123,43 ± 8,07 | 47,83 ± 2,03 |
| A"4 | 1,71 ± 0,19 | 88,18 ± 0,82 |
| | 8,35 ± 1,13 | 87,61 ± 0,84 |
| | 30,97 ± 2,98 | 81,14 ± 2,07 |
| | 54,40 ± 10,02 | 73,24 ± 3,46 |
| | 102,08 ± 14,70 | 57,02 ± 4,36 |
| | 119,90 ± 6,96 | 53,11 ± 3,46 |
| A'''4 | 1,69 ± 0,08 | 88,58 ± 0,32 |
| | 8,33 ± 0,30 | 87,66 ± 0,28 |
| | 31,65 ± 0,72 | 80,39 ± 0,89 |
| | 57,17 ± 3,81 | 71,01 ± 1,68 |
| | 104,64 ± 6,28 | 55,04 ± 0,68 |
| | 127,98 ± 4,83 | 49,77 ± 2,66 |

Les résultats indiqués dans ce tableau montrent que les pourcentages de [¹⁴C]warfarine liée à l'albumine varient en fonction de l'augmentation de la concentration en [¹⁴C]warfarine dans les trois compositions d'albumine considérées. Le site de fixation de l'albumine n'est donc pas saturé par le stabilisant.

La comparaison des résultats figurant aux Tableaux 10 et 11 démontre que l'albumine se comporte différemment vis-à-vis des médicaments considérés. Ceci s'expliquerait par la structure des médicaments considérés en rapport avec la configuration des sites de fixation de l'albumine. Ces études ont également permis de déterminer les différentes constantes de liaison (Ka) des deux médicaments sur les différentes albumines A'4, A"4 et A'''4, figurant au Tableau 12.

**Tableau 12**

| Composition d'albumine | Ka [¹⁴C] diazepam (mM⁻¹) | Ka [¹⁴C] warfarine (mM⁻¹) |
|---|---|---|
| A'4 | 187 | 215 |
| A"4 | 4,2 | 135 |
| A"'4 | 3,35 | 118 |

### Exemple 13

La capacité de stabilisation du facteur von Willebrand (fvW) par l'albumine nanofiltrée a été évaluée par des essais de chauffage à sec du fvW à 80°C pendant 72 heures. Sur la base d'essais préliminaires, il a été trouvé que l'ajout d'un mélange d'excipients judicieusement choisis tel que de glycine et d'arginine, à une solution de fvW, permettait sa stabilisation au cours du chauffage à sec (80°C, 72 heures). Bien que l'activité du fvW (fvW:Ag) était conservée (> 82%, par rapport au fvW non chauffé), son profil multimérique n'était en revanche pas maintenu, ce qui était observé par la disparition de multimères de hauts poids moléculaires (> 10), responsables de l'effet thérapeutique du fvW.

Pour pallier cet inconvénient, trois solutions de 1 g/l de fvW ont été préparées en présence de glycine et d'arginine, et d'albumine nanofiltrée A à usage thérapeutique. Les caractéristiques des solutions ainsi obtenues, A10, A11 et A12, figurent au Tableau 13.

**Tableau 13**

| Solutions | Albumine (g/l) | Glycine (g/l) | Arginine (g/l) |
|---|---|---|---|
| A10 | 15 | 5 | 40 |
| A11 | 15 | 10 | 40 |
| A12 | 15 | 5 | 30 |

Celles-ci sont ensuite soumises à une lyophilisation (45 ± 3 h) et à un chauffage à sec défini plus haut. Après le chauffage, l'ajout d'eau purifiée pour injection (PPI) dans les résidus secs, permet de reconstituer les solutions A11-A12. On procède à l'analyse des paramètres suivants : aspect de la solution, temps de dissolution, activité du fvW et profil multimérique, mesuré par électrophorèse. Le Tableau 14 présente les résultats obtenus.

**Tableau 14**

| Solutions | Aspect de la solution | Temps de dissolutio n (min) | fvW:Ag (%)** | Profil multimérique |
|---|---|---|---|---|
| A10 | L* | < 1 | 91 | C*** |
| A11 | L* | < 1 | 89 | C*** |
| A12 | L* | < 1 | 87 | C*** |

| | | | | |
|---|---|---|---|---|
| * : limpide, sans particules visibles ** : rapport entre l'activité du fvW chauffé et celle du fvW non chauffé *** : Conforme au produit de départ (avant chauffage) | | | | |

Ces résultats indiquent que la présence d'albumine non seulement n'induit pas d'effet négatif sur la stabilisation au chauffage du fvW, ce qui se traduit par un aspect limpide de solution (sans particules observables), un temps de solubilisation très faible et surtout, la conservation de l'activité du fvW, mais qu'elle permet également de conserver l'intégrité de ses multimères de hauts poids moléculaires.

## Revendications

1. Procédé de purification d'albumine **caractérisé en ce qu'**il comprend une étape consistant à soumettre une solution aqueuse d'albumine, de concentration 15 g/l à 80 g/l et de pH non inférieur à 7, à une nanofiltration dans une plage de température allant de 15°C à 55°C.

2. Procédé selon la revendication 1, **caractérisé en ce que** la nanofiltration est effectuée sur un filtre qualifié pour une porosité d'au moins 13 nm.

3. Procédé selon l'une des revendications 1 et 2, **caractérisé en ce que** le pH de la solution aqueuse d'albumine est situé dans la plage de valeurs allant de 7,8 à 11,5, et, de préférence, de 9 à 10,5.

4. Procédé selon l'une quelconque des revendications 1 à 3, **caractérisé en ce qu'**il comprend en outre une étape d'ajout d'un sel ou mélange de sels pharmaceutiquement acceptables dans la solution aqueuse d'albumine pour obtenir une solution de force ionique située dans la plage allant de 0,01 à 0,55.

5. Procédé selon la revendication 4, **caractérisé en ce que** le sel pharmaceutiquement acceptable est un sel d'un métal alcalin.

6. Procédé selon la revendication 5, **caractérisé en ce que** le sel d'un métal alcalin est le chlorure de sodium présent en quantité conférant à la solution d'albumine une force ionique de 0,15.

7. Procédé selon l'une quelconque des revendications 1. à 6, **caractérisé en ce que** la concentration de la solution aqueuse d'albumine est située dans la plage allant de 40 g/l à 60 g/l.

8. Procédé selon l'une quelconque des revendications 1 à 7, **caractérisé en ce que** la température de la solution aqueuse d'albumine est comprise entre 30°C et 55°C.

9. Procédé selon l'une quelconque des revendications 1 à 8, **caractérisé en ce que** la nanofiltration de la solution aqueuse d'albumine est effectuée en deux étapes successives sur respectivement deux filtres de porosité décroissante.

10. Procédé selon la revendication 9, **caractérisé en ce que** les deux étapes successives de nanofiltration sont effectuées respectivement sur des filtres de porosité de 23 à 50 nm et 15 ou 20 nm.

11. Procédé selon l'une quelconque des revendications 1 à 10, **caractérisé en ce qu'**il est mis en oeuvre avec des filtres en cellulose régénérée de 15 nm et de surface 0,01 m², à une pression non supérieure à 1 bar.

12. Procédé selon la revendication 11, **caractérisé en ce que** la pression est située dans la plage allant de 0,2 à 0,8 bar.

13. Procédé selon l'une quelconque des revendications 1 à 12, **caractérisé en ce que** l'albumine est obtenue par extraction éthanolique et/ou par purification par chromatographie d'échange d'ions ou d'affinité.

14. Procédé selon l'une quelconque des revendications 1 à 13, **caractérisé en ce qu'**il comprend une étape ultérieure de traitement d'adaptation de la solution aqueuse d'albumine à un usage thérapeutique.

15. Solution aqueuse d'albumine viralement sécurisée susceptible d'être obtenue par la mise en oeuvre du procédé selon l'une quelconque des revendications 1 à 14, dans laquelle les sites de transport et de fixation de principes actifs thérapeutiques de l'albumine sont disponibles.

16. Solution aqueuse d'albumine selon la revendication 15, **caractérisée en ce qu'**elle contient au plus 1% de polymères d'albumine de taille inférieure à 100 nm.

17. Solution aqueuse d'albumine selon la revendication 15 ou 16, **caractérisée en ce qu'**elle contient au plus 1% de polymères d'albumine de taille inférieure à 20 nm.

18. Composition d'albumine à usage thérapeutique obtenue par un traitement, selon la revendication 14, d'adaptation d'une solution aqueuse d'albumine, selon l'une quelconque des revendications 15 à 17, à un usage clinique.

19. Utilisation d'une composition d'albumine à usage thérapeutique selon la revendication 18, pour la stabilisation d'au moins un membre choisi dans le groupe constitué par les protéines de faible concentration et d'activité spécifique élevée, les immunoglobulines spécifiques, les anticorps monoclonaux, les vaccins, les allergènes, les cytokines et les hormones peptidiques.

20. Utilisation selon la revendication 19, **caractérisée en ce que** les protéines représentent le facteur VIII ou le facteur von Willebrand et leur équivalent recombinant.

21. Utilisation d'une composition d'albumine à usage thérapeutique selon la revendication 18, pour le transport et la fixation de principes actifs thérapeutiques.

22. Utilisation d'une composition d'albumine à usage thérapeutique selon la revendication 18, comme excipient pour un milieu d'incubation pour la fécondation "in vitro" d'ovocytes humains.

23. Utilisation d'une composition d'albumine à usage thérapeutique selon la revendication 18, comme protéine standard témoin.

## Claims

1. A method for purifying albumin, **characterized in that** it comprises a step consisting in subjecting an aqueous solution of albumin, having a concentration of from 15 g/l to 80 g/l and a pH of not less than 7, to nanofiltration in a temperature range from 15°C to 55°C.

2. A method according to claim 1, **characterized in that** the nanofiltration is carried out on a filter defined for a porosity of at least 13 nm.

3. A method according to either of claims 1 and 2, **characterized in that** the pH of the aqueous solution of albumin is within the range of values from 7.8 to 11.5, and preferably from 9 to 10.5.

4. A method according to any one of claims 1 to 3, **characterized in that** it also comprises a step of addition of a pharmaceutically acceptable salt or mixture of pharmaceutically acceptable salts to the aqueous solution of albumin so as to obtain a solution with an ionic strength within the range of from 0.01 to 0.55.

5. A method according to claim 4, **characterized in that** the pharmaceutically acceptable salt is a salt of an alkali metal.

6. A method according to claim 5, **characterized in that** the salt of an alkali metal is sodium chloride present in an amount that confers on the solution of albumin an ionic strength of 0.15.

7. A method according to any one of claims 1 to 6, **characterized in that** the concentration of the aqueous solution of albumin is within the range of from 40 g/l to 60 g/l.

8. A method according to any one of claims 1 to 7, **characterized in that** the temperature of the aqueous solution of albumin is from 30°C to 55°C.

9. A method according to any one of claims 1 to 8, **characterized in that** the nanofiltration of the aqueous solution of albumin is carried out in two successive steps on respectively two filters of decreasing porosity.

10. A method according to claim 9, **characterized in that** the two successive nanofiltration steps are carried out respectively on filters with a porosity of from 23 to 50 nm and 15 or 20 nm.

11. A method according to any one of claims 1 to 10, **characterized in that** it is carried out with 15-nm regenerated cellulose filters having a surface area of 0.01 m², at a pressure no greater than 1 bar.

12. A method according to claim 11, **characterized in that** the pressure is within the range from 0.2 to 0.8 bar.

13. A method according to any one of claims 1 to 12, **characterized in that** the albumin is obtained by ethanolic extraction and/or by purification by ionexchange or affinity chromatography.

14. A method according to any one of claims 1 to 13, **characterized in that** it comprises a subsequent step of treatment for adapting the aqueous solution of albumin to therapeutic use.

15. A virally safe aqueous solution of albumin that can be obtained by carrying out the method according to any one of claims 1 to 14, in which the sites for the transport and binding of therapeutic active ingredients of the albumin are available.

16. An aqueous solution of albumin according to claim 15, **characterized in that** it contains at most 1% of albumin polymers less than 100 nm in size.

17. An aqueous solution of albumin according to claim 15 or 16, **characterized in that** it contains at most 1% of albumin polymers less than 20 nm in size.

18. An albumin composition for therapeutic use, obtained by a treatment, according to claim 14, for adapting an aqueous solution of albumin, according to any one of claims 15 to 17, to clinical use.

19. The use of an albumin composition for therapeutic use according to claim 18, for stabilizing at least one member chosen from the group consisting of proteins of low concentration and high specific activity, specific immunoglobulins, monoclonal antibodies, vaccines, allergens, cytokines and peptide hormones.

20. The use according to claim 19, **characterized in that** the proteins represent factor VIII or von Willebrand factor and their recombinant equivalent.

21. The use of an albumin composition for therapeutic use according to claim 18, for the transport and binding of therapeutic active ingredients.

22. The use of an albumin composition for therapeutic use according to claim 18, as an excipient for an incubation medium for "in vitro" fertilization of human ovocytes.

23. The use of an albumin composition for therapeutic use according to claim 18, as a control standard protein.

## Patentansprüche

1. Verfahren zur Reinigung von Albumin, **dadurch gekennzeichnet, daß** es eine Stufe umfaßt, die aus dem Unterziehen einer wäßrigen Albuminlösung mit einer Konzentration von 15 g/l bis 80 g/l und einem pH nicht unter 7 einer Nanofiltration in einem Temperaturbereich von 15 °C bis 55 °C besteht.

2. Verfahren gemäß Anspruch 1, **dadurch gekennzeichnet, daß** die Nanofiltration auf einem Filter ausgeführt wird, das sich durch eine Porosität von mindestens 13 nm auszeichnet.

3. Verfahren gemäß einem der Ansprüche 1 und 2, **dadurch gekennzeichnet, daß** der pH der wäßrigen Albuminlösung zwischen Werten von 7,8 bis 11,5 und bevorzugt von 9 bis 10,5 liegt.

4. Verfahren gemäß einem der Ansprüche 1 bis 3, **dadurch gekennzeichnet, daß** es außerdem eine Stufe des Zufügens eines pharmazeutisch annehmbaren Salzes oder Gemischs pharmazeutisch annehmbarer Salze zu der wäßrigen Albuminlösung umfaßt, um eine Lösung mit einer zwischen 0,01 und 0,55 liegenden Ionenstärke zu erhalten.

5. Verfahren gemäß Anspruch 4, **dadurch gekennzeichnet, daß** das pharmazeutisch annehmbare Salz ein Alkalimetallsalz ist.

6. Verfahren gemäß Anspruch 5, **dadurch gekennzeichnet, daß** das Alkalimetallsalz Natriumchlorid ist, das in einer Menge vorliegt, um der Albuminlösung eine Ionenstärke von 0,15 zu verleihen.

7. Verfahren gemäß einem der Ansprüche 1 bis 6, **dadurch gekennzeichnet, daß** die Konzentration der wäßrigen Albuminlösung zwischen 40 g/l und 60 g/l liegt.

8. Verfahren gemäß einem der Ansprüche 1 bis 7, **dadurch gekennzeichnet, daß** die Temperatur der wäßrigen Albuminlösung zwischen 30 °C und 55 °C liegt.

9. Verfahren gemäß einem der Ansprüche 1 bis 8, **dadurch gekennzeichnet, daß** die Nanofiltration der wäßrigen Albuminlösung in zwei aufeinanderfolgenden Stufen auf zwei Filtern mit abnehmender Porosität ausgeführt wird.

10. Verfahren gemäß Anspruch 9, **dadurch gekennzeichnet, daß** die beiden aufeinanderfolgenden Stufen der Nanofiltration auf Filtern der Porosität 23 bis 50 nm beziehungsweise 15 oder 20 nm ausgeführt werden.

11. Verfahren gemäß einem der Ansprüche 1 bis 10, **dadurch gekennzeichnet, daß** es mit Filtern aus regenerierter Cellulose mit 15 nm und 0,01 m² Oberfläche bei einem Druck nicht über 1 bar durchgeführt wird.

12. Verfahren gemäß Anspruch 11, **dadurch gekennzeichnet, daß** der Druck zwischen 0,2 und 0,8 bar liegt.

13. Verfahren gemäß einem der Ansprüche 1 bis 12, **dadurch gekennzeichnet, daß** das Albumin durch Ethanolextraktion und/oder durch Reinigung durch Ionenaustausch- oder Affinitätschromatographie erhalten wird.

14. Verfahren gemäß einem der Ansprüche 1 bis 13, **dadurch gekennzeichnet, daß** es eine weitere Behandlungsstufe zur Anpassung der wäßrigen Albuminlösung an eine therapeutische Verwendung umfaßt.

15. Virussichere wäßrige Albuminlösung, erhältlich durch Ausführen eines Verfahrens gemäß einem der Ansprüche 1 bis 14, bei der die Stellen zum Transport und Fixierung therapeutischer Wirkstoffe des Albumins zur Verfügung stehen.

16. Wäßrige Albuminlösung gemäß Anspruch 15, **dadurch gekennzeichnet, daß** sie höchstens 1 % Albuminpolymere mit einer Größe unter 100 nm enthält.

17. Wäßrige Albuminlösung gemäß einem der Ansprüche 15 oder 16, **dadurch gekennzeichnet, daß** sie höchstens 1 % Albuminpolymere mit einer Größe unter 20 nm enthält.

18. Albuminzusammensetzung zur therapeutischen Verwendung, die durch eine Anpassungsbehandlung gemäß Anspruch 14 einer wäßrigen Albuminlösung gemäß einem der Ansprüche 15 bis 17 an eine klinische Verwendung erhalten wurde.

19. Verwendung einer Albuminzusammensetzung zur therapeutischen Verwendung gemäß Anspruch 18 zur Stabilisierung mindestens eines Elements, das aus der aus Proteinen mit geringer Konzentration und erhöhter spezifischer Aktivität, spezifischen Immunglobulinen, monoklonalen Antikörpern, Vakzinen, Allergenen, Zytokinen und Peptidhormonen bestehenden Gruppe ausgewählt ist.

20. Verwendung gemäß Anspruch 19, **dadurch gekennzeichnet, daß** die Proteine den Faktor VIII oder den Von-Willebrand-Faktor und ihr rekombinantes Äquivalent bedeuten.

21. Verwendung einer Albuminzusammensetzung zur therapeutischen Verwendung gemäß Anspruch 18 zum Transport und der Fixierung therapeutischer Wirkstoffe.

22. Verwendung einer Albuminzusammensetzung zur therapeutischen Verwendung gemäß Anspruch 18 als Träger für ein Inkubationsmedium zur In-vitro-Fertilisation menschlicher Eizellen.

23. Verwendung einer Albuminzusammensetzung zur therapeutischen Verwendung gemäß Anspruch 18 als Proteinvergleichsstandard.
